(19) Europäisches Patentamt
European Patent Office
Office européen des brevets

(11) Veröffentlichungsnummer : **0 053 696**
**B1**

(12) # EUROPÄISCHE PATENTSCHRIFT

(45) Veröffentlichungstag der Patentschrift : **18.09.85**

(51) Int. Cl.⁴ : **C 07 C 85/06**, C 07 C 85/24

(21) Anmeldenummer : **81108813.7**

(22) Anmeldetag : **23.10.81**

(54) **Verfahren zur Herstellung von cycloaliphatischen und/oder aromatischen Aminen.**

(30) Priorität : **04.12.80 DE 3045719**

(43) Veröffentlichungstag der Anmeldung : **16.06.82 Patentblatt 82/24**

(45) Bekanntmachung des Hinweises auf die Patenterteilung : **18.09.85 Patentblatt 85/38**

(84) Benannte Vertragsstaaten : **AT CH DE FR GB IT LI NL SE**

(56) Entgegenhaltungen :
**EP-A- 0 022 751**
**CH-A- 445 476**
**DE-B- 2 045 882**
**FR-A- 1 506 010**
**US-A- 3 351 661**
**US-A- 3 361 818**
**US-A- 3 931 298**

(73) Patentinhaber : **BASF Aktiengesellschaft**
**Carl-Bosch-Strasse 38**
**D-6700 Ludwigshafen (DE)**

(72) Erfinder : **Goetz, Norbert, Dr.**
**Schoefferstrasse 25**
**D-6520 Worms (DE)**
Erfinder : **Jacobs, Peter, Dr.**
**Kalmitstrasse 2**
**D-6718 Gruenstadt (DE)**
Erfinder : **Hupfer, Leopold, Dr.**
**Waltershoehe 3**
**D-6701 Friedelsheim (DE)**
Erfinder : **Toussaint, Herbert, Dr.**
**Knietschstrasse 11**
**D-6710 Frankenthal (DE)**
Erfinder : **Reiss, Wolfgang, Dr.**
**Bannwasserstrasse 70**
**D-6700 Ludwigshafen (DE)**

**EP 0 053 696 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von Aminen aus entsprechenden aromatischen Hydroxyverbindungen (Phenolen).

Die älteste und zur Zeit immer noch wichtigste Methode zur Herstellung primärer aromatischer Amine ist die Reduktion der entsprechenden Nitroverbindungen (Houben-Weyl « Methoden der Org. Chemie », Band 11/1, S. 360 ff). Nachteilig ist dabei die Tatsache, daß die Nitrierung bei substituierten Aromaten oft nicht einheitlich verläuft und fast stets Isomerengemische anfallen.

Da Phenole im allgemeinen gut in reiner Form zugängliche Verbindungen sind, liegt es nahe, entsprechend dem Syntheseweg zur Herstellung aliphatischer Amine die Hydroxygruppe gegen eine Aminogruppe auszutauschen, um so zu Anilinen mit einheitlichem Substitutionsmuster zu gelangen.

Prinzipiell gibt es die Möglichkeit, Phenole durch Partialhydrierung in Cyclohexanone zu überführen (DE-AS 1 124 487, DE-AS 1 298 098, DE-AS 1 144 267, US-PS 3 124 614, CH-PS 463 493, DE-OS 2 045 882), die Cyclohexanone mit Ammoniak und Wasserstoff zu Cyclohexylaminen umzusetzen (Houben-Weyl, loc. cit., S. 611-617) und zu aromatischen Aminen zu dehydrieren (US-PS 3 361 818) oder die Cyclohexanone mit Ammoniak direkt in die aromatischen Amine umzuwandeln (US-PS 3 219 702, 3 219 704).

Diese Wege sind aber, da sie mehrere unabhängige Verfahrensschritte erfordern, recht umständlich und nicht besonders wirtschaftlich.

Die einstufige (direkte) Überführung von Phenolen mit Ammoniak und Wasserstoff in Cyclohexylamine gelingt in Gegenwart von teueren Ruthenium- oder Rhodiumkatalysatoren (JA-OS 4 034 677, FR-PS 1 427 543, GB-PS 1 031 169). Eine Methode zur direkten Herstellung von aromatischen Aminen aus Phenolen ist ebenfalls bereits beschrieben worden (DE-OS 2 208 827, US-PSen 3 931 298, 3 960 962). Bei diesem Verfahren soll der Ablauf der Reaktion die Gegenwart einer Cyclohexanonverbindung voraussetzen, die dem Reaktionsgemisch als Katalysator anfangs in einer gewissen Menge zugesetzt oder aus dem Phenol erzeugt werden muß. Man kann vermuten, daß es sich bei diesem Verfahren um eine unmittelbare Aneinanderreihung der oben erwähnten Verfahrensschritte handelt, die über ein Cyclohexanon als Zwischenprodukt verlaufen. Als Katalysator soll bei diesem Verfahren außer dem Cyclohexanon ein Hydrierkatalysator verwendet werden, wobei Palladium bevorzugt zu sein scheint.

Das europäische Patent Nr. 53 817 beschreibt ein Verfahren zur Herstellung von cycloaliphatischen Aminen durch Umsetzung von entsprechenden Phenolen mit Ammoniak und Wasserstoff bei erhöhter Temperatur, wobei man die Umsetzung in Gegenwart eines auf einem Träger aufgebrachten Ruthenium-, Rhodium-, Palladium- oder Platin-Katalysators durchführt und einen Wasserstoffpartialdruck von wenigstens 20 bar bzw. einen Gesamtdruck von wenigstens 50 bar einhält.

Gegenstand des europäischen Patents Nr. 53 819 ist ein Verfahren zur Herstellung von cycloaliphatischen und/oder aromatischen Aminen durch Umsetzung von entsprechenden Phenolen mit Ammoniak und Wasserstoff, wobei man die Umsetzung in Gegenwart eines auf einem Träger aufgebrachten Ruthenium-, Rhodium- oder Platin-Katalysators, der einen basischen Stoff und/oder ein Element aus der Gruppe 1B, 2B, 7B des Periodensystems, Eisen, Kobalt oder Nickel enthält, in der Gasphase durchführt.

Es wurde nun gefunden, daß man ein- oder mehrkernige, cycloaliphatische und/oder aromatische Amine zum Beispiel der allgemeinen Formel I, Ia

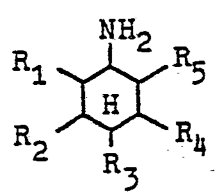

bzw.

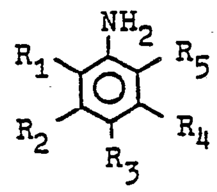

(I, Ia)

in der $R_1$ bis $R_5$ jeweils ein Wasserstoffatom oder gleiche oder verschiedene oder jeweils zu mehreren gemeinsam einen Substituenten bedeuten können, in guten Ausbeuten erhält, wenn man ein entsprechendes Phenol mit Ammoniak und Wasserstoff bei einer Temperatur zwischen 100 und 400 °C an einem Palladium-Katalysator umsetzt, der einen basischen Stoff und/oder ein Element aus der Gruppe 1b, 2b, 7b des Periodensystems, Eisen, Kobalt oder Nickel enthält, wobei die Herstellung von cycloaliphatischen Verbindungen unter Verwendung von einem Wasserstoffpartialdruck von wenigstens 20 bar bzw. einem Gesamtdruck von wenigstens 50 bar gemäß des europäischen Patents Nr. 53 817 ausgenommen wird.

Aliphatische Gruppen mit 1 bis 20 Kohlenstoffatomen, cycloaliphatische Gruppen mit 5 bis 20 Kohlenstoffatomen, Arylgruppen mit 6 bis 20 Kohlenstoffatomen oder Aralkyl- oder Arylalkylgruppen mit 7 bis 20 Kohlenstoffatomen sind Beispiele für derartige Substituenten. Diese können gegebenenfalls Sauerstoff und/oder Stickstoff als Heteroatome enthalten oder es können zwei Substituenten durch eine Molekülbrücke miteinander verbunden sein.

Mit den erfindungsgemäßen Katalysatoren erhält man je nach Reaktionstemperatur entweder

überwiegend cycloaliphatische oder aromatische Amine, und zwar mit ausgezeichneter Selektivität und Reinheit; das heißt, daß die Bildung von Nebenprodukten fast vollständig unterdrückt werden kann. Dieser Befund ist überraschend, da nach dem Stande der Technik bei höherer Temperatur zahlreiche Nebenreaktionen und somit Nebenprodukte, wie Cyclohexanol, Cyclohexanon, Dicyclohexylamin, Phenyl-cyclohexylamin und Diphenylamin zu erwarten sind. Durch Wahl der Bedingungen kann der Reaktionsablauf so gesteuert werden, daß ausschließlich gesättigte Cyclohexylamine oder überwiegend entsprechende aromatische Amine (Aniline) gebildet werden : Während z. B. bei atmosphärischem Druck die entsprechenden aromatischen Amine begünstigt sind, ist ein Betrieb unter Druck zu bevorzugen, wenn die cycloaliphatischen Amine erhalten werden sollen. Temperatursteigerung begünstigt die Bildung der Aniline, Erhöhung des Wasserstoffdruckes wirkt im Sinne einer Bevorzugung der Cyclohexylamine, so daß im unteren Temperaturbereich (150-230 °C) in erster Linie die gesättigten Amine, im oberen Temperaturbereich (180-300 °C) hauptsächlich Aniline anfallen. Die auftretenden Cyclohexylamine lassen sich überdies bei Bedarf leicht in Aniline überführen (Beispiel 5b, 10), umgekehrt ist es aber auch möglich, durch Hydrierung die aromatischen Amine in die Cyclohexylamine umzuwandeln (Beispiel 11). Cyclohexylamine und entsprechende Aniline lassen sich im übrigen leicht trennen. Auf dieser Tatsache beruht der wesentliche Vorteil des hier beschriebenen Verfahrens, denn die als Ausgangsstoffe verwendeten Phenole lassen sich dabei vollständig umsetzen, so daß die aufwendige und teuere Trennung von Phenol und Anilin vermieden wird, wie sie z. B. bei der Methode der DE-OS 2 208 827 notwendig ist.

Die als Ausgangsstoffe verwendeten Phenole sind im allgemeinen gut zugängliche Verbindungen (Houben-Weyl, Methoden, Band 6/1c).

Entsprechend der o. a. Formel für die Zielprodukte kann ein entsprechendes Phenol Substituenten $R_1$ bis $R_5$ haben, von denen einer oder mehrere auch selbst ein Phenol darstellen können, so daß also unter dem Begriff Phenol ein- oder mehrkernige Phenole zu verstehen sind. Bisphenol A ist ein Beispiel für ein mehrkerniges Phenol.

$R_1$ bis $R_5$ können ferner gleiche oder verschiedene Substituenten und selbstverständlich Wasserstoff bedeuten, wobei Alkyl- oder Aralkylreste mit jeweils 1 bis 20 C-Atomen Kettenlänge besonders wichtige Substituenten darstellen. Die Substituenten können außerdem Sauerstoff- oder Stickstoffatome in der Kette oder in heterocyclischer Anordnung aufweisen. Benachbarte Substituenten können zusammen mit dem Phenolrest einen Ring bilden, so daß als Phenole auch Naphthole und teilweise hydrierte Naphthole zu gelten haben.

Zwar ist ist die Umsetzung von Hydroxybenzol (« Phenol ») selbst nicht wirtschaftlich interessant, aber sie ist technisch möglich. Andere, substituierte Phenole sind etwa o-, m- und p-Kresol, o-Ethylphenol, o-n-Butylphenol, o-sek.-Butylphenol, 2,4-Dimethylphenol, 2,6-Dimethylphenol, 2,3,6-Trimethylphenol, 2,4,6-Trimethylphenol, 2-Cyclohexylphenol, 2,6-Dimethyl-3-phenyl-phenol, 2,6-Diethylphenol, 2,5-Diisopropylphenol, 2,6-Di-tert.-butylphenol, 2-Methyl-6-sek.-butylphenol, 3-tert.-Butylphenol, α-Natphthol, β-Naphthol, Bisphenol A (= 2,2-Di-(p-Hydroxyphenyl)-propan).

Der erforderliche Ammoniak kann — bezogen auf das eingesetzte Phenol — in stöchiometrischer Menge oder in einem, auch hohen Überschuß angewendet werden.

Das Verfahren kann kontinuierlich (fortlaufend) oder diskontinuierlich (absatzweise) in der Gasphase oder Flüssigphase durchgeführt werden. In jedem Falle kann der Katalysator in einem Festbett oder als Wirbelbett bzw. Suspension angeordnet sein. Bevorzugt bei kontinuierlicher Arbeitsweise ist ein fest angeordneter Katalysator. Bei kontinuierlicher Arbeitsweise wird der Ammoniak zusammen mit dem Wasserstoff in der Regel im Verhältnis von 10 : 1 bis 1 : 10 über den Katalysator geleitet. Im Falle diskontinuierlichen Betriebs führt man so lange Wasserstoff zu, bis das eingesetzte Phenol vollständig umgesetzt ist. Die Umsetzung kann ohne Lösungsmittel oder in Anwesenheit von Lösungsmitteln, die unter den Reaktionsbedingungen chemisch indifferent sind, durchgeführt werden.

Als Lösungsmittel kommen beispielsweise in Frage : Methanol, Ethanol, n-Butanol, Tetrahydrofuran, Dioxan, Cyclohexyl-methylether, Methylglykol, Ethylglykol, 1,2-Dimethoxyethan, N,N-Dimethylcyclohexylamin, N-Methylpiperidin, N-Ethylpiperidin, N-Methylmorpholin, N-Methylpyrrolidin, Cyclohexan. In vielen Fällen ist das Zielprodukt selbst ein günstiges Lösungsmittel ; man führt einen Teilstrom zurück, wenn eine entsprechende Verdünnung gewünscht wird.

Das nach dem Verfahren der Erfindung verwendete Katalysatorsystem enthält einerseits Palladium und andererseits aktive Zusätze, wobei die Zusätze allein oder im Gemisch miteinander neben dem Palladium vorliegen können. Die aktiven Bestandteile des Katalysators sind auf einem Träger aufgebracht, der aus Aluminiumoxid, Kieselsäure, Aluminiumsilikat, Magnesiumsilikat, Aktivkohle oder Spinellen des Aluminiums, Chroms oder Eisens bestehen kann, wobei die Zusätze, die dem Katalysator seine Eigenschaften verleihen, sich an der Oberfläche des Katalysators befinden können, zusammen mit einem chemisch indifferenten Material in Form einer Mischung den Träger darstellen können oder mit dem ursprünglichen Träger durch nachträgliches Tempern ein gemeinsames Kristallgitter bilden können (beispielsweise bei Aluminiumoxidträgern Bildung von typischen Spinellgittern mit bestimmten Metallen). Das bedeutet, daß der Träger Aktivität und auch Lebensdauer des Katalysators günstig beeinflussen kann. Vorzugsweise wird Aluminiumoxid als Träger verwendet.

Als Zusätze kommen in Frage :

a) Basische Zusätze, wie Oxide, Hydroxide oder Carbonate der Alkalimetalle, vorzugsweise des

Lithiums und Natriums, der Erdalkalimetalle, vorzugsweise Magnesium und Calcium und der Seltenerdmetalle, vorzugsweise Cer und Praseodym (Neodym).

b) Weitere Metalle, wie Nickel, Cobalt, Mangan, Zink, Cadmium und Silber.

Die Zusätze können gemeinsam mit dem Palladium durch Tränken des Trägermaterials mit Lösungen von z. B. Nitraten, Chloriden, Formiaten oder Oxalaten aufgebracht werden. Durch anschließende thermische Behandlung, die gewöhnlich zwischen 400 und 600 °C durchgeführt wird, erfolgt Überführung in die Oxidform. Soll bei Aluminiumoxidträgern mit geeigneten Metallen (Mg, Zn, Co, Mn, Li) Spinellbildung erzielt werden, muß nach der Tränkung auf eine Temperatur von 900-1 300 °C erhitzt werden (siehe Ullmanns Encyklopädie der technischen Chemie, 3. Auflage (1955) Band 6, S. 242-244, Gmelin, System-Nr. 35, Al, Tl, 1934-1935, S. 26-28) und anschließend in der üblichen Weise das Palladium aufgebracht werden. Manche Zusätze, wie beispielsweise Calciumoxid oder Magnesiumoxid lassen sich mit einem Träger wie Aluminiumoxid mischen und bilden dann nach gemeinsamem Tempern bei 400-600 °C einen neuen Träger, auf dem das Palladium niedergeschlagen werden kann. Lösliche Zusatzstoffe, wie Natriumcarbonat, können getrennt vom eigentlichen Hydrierkatalysator in Form ihrer Lösungen dem umzusetzenden Produkt zugeführt werden.

Der Palladiumgehalt des Katalysators, bezogen auf das Trägermaterial, liegt gewöhnlich zwischen 0,05 bis 15 Gew.%. Das Gewichtsverhältnis der Zusätze zu Palladium kann unterschiedlich sein ; es kann z. B. zwischen 10 000 : 1 bis 1 : 50, bevorzugt bei 100 : 1 bis 1 : 50 liegen. Man verwendet den Katalysator beispielsweise in Form von Strängen mit einem Durchmesser von 3 mm und einer Länge von 10 mm oder als Pulver, je nach dem vorgesehenen Anwendungszweck.

Die nach dem Verfahren der Erfindung hergestellten Verbindungen werden z. B. als Zwischenprodukte zur Herstellung von Wirkstoffen in Pflanzenschutzmitteln verwendet (DAS 2 305 495, DOS 2 648 008, DOS 2 513 732, DOS 2 515 091).

## Herstellmöglichkeiten für den Katalysator

a) Auf strang- oder pulverförmiges $\gamma$-Aluminiumoxid wird Palladium zusammen mit dem Zusatz (Mangan, Zink, Silber, Seltenerdmetalle u. a.) in der gewünschten Menge durch Tränken mit beispielsweise deren Nitratlösungen und anschließendes Eindampfen bis zur Trockne aufgebracht. Danach wird 6 Stunden bei 550 °C getempert und im Wasserstoffstrom bei 300 °C reduziert.

b) Auf strang- oder pulverförmiges $\gamma$-Aluminiumoxid wird zunächst die gewünschte Menge des Zusatzes (Mangan, Zink, Cobalt, Magnesium, Lithium u. a.) durch Tränken mit entsprechenden wäßrigen Nitrat- oder Formiatlösungen aufgebracht und bei 150 °C getrocknet. Der derart vorbehandelte Träger wird nun entweder 6 Stunden lang auf 550 °C erhitzt oder, wenn Spinellbildung erreicht werden soll, 6 Stunden lang bei 1 050 °C geglüht. Nun wird der Träger mit wäßriger Palladiumnitratlösung getränkt und durch 7-stündiges Erhitzen bei 300 °C im Wasserstoffstrom reduziert. Wurde zum Tränken Palladium-(II)-chloridlösung genommen, wird mit alkalischer Formalinlösung reduziert.

c) $\gamma$-Aluminiumoxid und ein basisches Oxid (MgO, CaO) werden in dem gewünschten Mengenverhältnis intensiv miteinander vermischt. Diese Mischung wird 6 Stunden lang auf 450 °C erhitzt, anschließend mit Palladiumnitratlösung getränkt und 7 Stunden mit Wasserstoff bei 300 °C reduziert. Der Katalysator wird mit einer 5 %igen wäßrigen Hydrazinhydratlösung nachreduziert und dann bei 120 °C getrocknet.

## Beispiel 1

In ein druckfestes zylindrisches Rohr mit einem Volumen von 1,2 l als Reaktor wird ein Katalysator in Form von Strängen (3 mm Durchmesser, 10 mm Länge), der 0,5 Gew.% Palladium auf einer Mischung aus 19,4 Gew.% Magnesiumoxid und 80,6 Gew.% Aluminiumoxid enthält, eingefüllt und auf 220 °C erhitzt. Hierüber werden stündlich bei atmosphärischem Druck 100 g 2,6-Dimethylphenol geführt. Gleichzeitig wird eine Gasmischung aus 250 Nl Ammoniak und 250 Nl Wasserstoff pro Stunde im Gleichstrom hindurchgeleitet. Das Reaktionsprodukt wird — sobald es den Reaktor verläßt — abgekühlt. Es besteht nach gaschromatographischer Analyse aus 78 Gew.% 2,6-Dimethylanilin ($Kp_{760}$ = 216 °C) und 22 Gew.% 2,6-Dimethylcyclohexylamin ($Kp_{760}$ = 167-168 °C). Die beiden Amine können durch Destillation leicht getrennt werden ; das Cyclohexylamin kann entweder in die Reaktionszone zurückgeführt oder separat zum 2,6-Dimethylanilin dehydriert werden (siehe Beispiele 5b, 10). Bei vollständiger Überführung des eingesetzten 2,6-Dimethylphenols in 2,6-Dimethylanilin erhält man aus je 100 g 2,6-Dimethylphenol 93,5 g 2,6-Dimethylanilin, entsprechend einer Ausbeute von 94 % der berechneten Ausbeute.

## Beispiel 2

Man verfährt entsprechend Beispiel 1, verwendet aber einen Katalysator, der 0,5 Gew.% Palladium, 19,4 Gew.% Calciumoxid und 80,6 Gew.% Aluminiumoxid enthält. Ausgehend von m-tert.-Butylphenol erhält man m-tert.-Butylanilin ($Kp_{0,2}$ = 72-73 °C) in einer Ausbeute von 96 %.

Beispiel 3

Man verfährt entsprechend Beispiel 1, verwendet aber einen Katalysator, der 1,0 Gew.% Palladium auf einem Kobalt-Aluminiumspinell enthält. Aus Phenol wird Anilin (Kp = 184 °C) in einer Ausbeute von 96 % erhalten.

Beispiel 4

Man verfährt entsprechend Beispiel 1, verwendet aber einen Katalysator, der 1,0 Gew.% Palladium auf einem Lithium-Aluminiumspinell enthält. Ausgehend von 2-Ethylphenol wird 2-Ethylanilin (Kp = 210 °C) in einer Ausbeute von 93 % erhalten.

Beispiel 5

In einem 300 ml fassenden Rührautoklaven wird eine Mischung aus 54,5 g α-Naphthol, 41 g flüssigem Ammoniak und 6 g eines Katalysators, der 5,0 Gew.% Palladium, 1,0 Gew.% Mangan und 5,0 Gew.% Silber, Rest Aluminiumoxid, enthält, bei 275 °C und 250 bar Wasserstoffdruck 30 Stunden lang bei konstantem Druck hydriert. Damit wird α-Naphthol zu α-Naphthylamin (Fp. 49 °C) mit einer Selektivität von 92 % d. Th. umgesetzt.

Beispiel 6

Man verfährt wie in Beispiel 5 beschrieben, verwendet aber einen Katalysator, der 10 Gew.% Palladium, 0,11 Gew.% Zink und 0,10 Gew.% Cadmium, Rest Aluminiumoxid, enthält. Damit wird 2,6-Dimethyl-3-phenyl-phenol zu 2,6-Dimethyl-3-phenylanilin ($Kp_{0,15}$ = 121 °C) mit einer Selektivität von 91 % umgesetzt.

Beispiel 7

Man verfährt wie in Beispiel 5 beschrieben, verwendet aber einen Katalysator, der 5,0 Gew.% Palladium und 2,5 Gew.% Cer-IV-oxid, Rest Aluminiumoxid, enthält und wählt eine Reaktionstemperatur von 250 °C. Damit wird 2,6-Dimethyl-3-(p-methoxyphenyl)-phenol zu 2,6-Dimethyl-3-(p-methoxyphenyl)-anilin ($Kp_{0,3}$ = 151 °C) mit einer Selektivität von 85 % umgesetzt.

Beispiel 8

In ein zylindrisches Rohr mit einem Volumen von 1,2 l wird ein Katalysator in Form von Strängen (3 mm Durchmesser, 10 mm Länge) aus 0,5 Gew.% Palladium, 19,4 Gew.% Magnesiumoxid und 80,6 Gew.% Aluminiumoxid eingefüllt und auf 160 °C erhitzt. Über dieses Katalysatorbett werden stündlich bei Normaldruck 100 g 2,6-Dimethylphenol geführt. Gleichzeitig wird ein Gasgemisch aus 200 Nl Ammoniak und 300 Nl Wasserstoff pro Stunde im Gleichstrom durch das Reaktionsrohr hindurchgeleitet. Das Reaktionsprodukt wird abgekühlt. Es besteht nach gaschromatographischer Analyse aus 94 Gew.% 2,6-Dimethylcyclohexylamin und 6 Gew.% 2,6-Dimethylanilin.

Beispiel 9

Man verfährt entsprechend Beispiel 8, verwendet aber als Ausgangsprodukt 2,6-Dimethyl-3-phenyl-phenol. Als Endprodukt wird 2,6-Dimethyl-3-phenyl-cyclohexylamin ($Kp_{0,1}$ = 103-105 °C) in einer Ausbeute von 92 % erhalten.

Beispiel 10

In ein druckfestes zylindrisches Rohr mit einem Volumen von 1,2 l als Reaktor wird ein Katalysator in Form von Strängen (3 mm Durchmesser, 10 mm Länge), der 0,5 Gew.% Palladium auf einer Mischung aus 19,4 Gew.% Magnesiumoxid und 80,6 Gew.% Aluminiumoxid enthält, eingefüllt und auf 130 °C erhitzt. Hierüber werden stündlich bei Atmosphärendruck 100 g 2,6-Dimethylphenol geführt. Gleichzeitig wird eine Gasmischung aus 250 Nl Ammoniak und 250 Nl Wasserstoff pro Stunde im Gleichstrom hindurchgeleitet. Das Reaktionsprodukt wird — sobald es den Reaktor verläßt — abgekühlt. Es besteht nach gaschromatographischer Analyse aus 96,4 Gew.% 2,6-Dimethylcyclohexylamin ($Kp_{760}$ = 167-168 °C) und 3,6 Gew.% 2,6-Dimethylanilin ($Kp_{760}$ = 216 °C). Die beiden Amine können durch Destillation leicht voneinander getrennt werden. Aus 100 g 2,6-Dimethylphenol erhält man auf diese Weise 97,5 g 2,6-Dimethylcyclohexylamin, entsprechend 93,5 % der berechneten Ausbeute.

**Patentansprüche**

1. Verfahren zur Herstellung von cycloaliphatischen und/oder aromatischen Aminen der allgemeinen Formel $RNH_2$, in der R einen gegebenenfalls substituierten oder annellierten Aryl- oder Cycloalkylrest bedeutet, durch Umsetzung von entsprechenden aromatischen Hydroxyverbindungen (Phenolen) mit Ammoniak und Wasserstoff in Gegenwart eines Katalysators bei erhöhter Temperatur und vorzugsweise erhöhtem Druck, dadurch gekennzeichnet, daß man die Umsetzung in Gegenwart eines auf einem Träger aufgebrachten Palladiumkatalysators durchführt, der einen basischen Stoff und/oder ein Element aus der Gruppe 1B, 2B, 7B des Periodensystems, Eisen, Kobalt oder Nickel enthält, wobei die Herstellung von cycloaliphatischen Verbindungen unter Verwendung von einem Wasserstoffpartialdruck von wenigstens 20 bar bzw. einem Gesamtdruck von wenigstens 50 bar gemäß des europäischen Patents Nr. 53 817 ausgenommen wird.

2. Verfahren gemäß Anspruch 1, dadurch gekennzeichnet, daß der Katalysator als basischen Stoff ein Oxid, Hydroxid oder Carbonat eines Alkalimetalls oder eines Erdalkalimetalls enthält.

3. Verfahren gemäß Anspruch 1, dadurch gekennzeichnet, daß der Katalysator als basischen Stoff ein Oxid, Hydroxid oder Carbonat eines Seltenerdmetalls enthält.

4. Verfahren nach Anspruch 2, dadurch gekennzeichnet, daß der Katalysator eine Magnesiumverbindung enthält.

5. Verfahren nach Anspruch 3, dadurch gekennzeichnet, daß der Katalysator eine Praseodym/Neodym- oder Cerverbindung enthält.

**Claims**

1. A process for the preparation of a cycloaliphatic and/or aromatic amine of the general formula $RNH_2$, where R is an optionally substituted or fused aryl or cycloalkyl radical, by reacting a corresponding aromatic hydroxy compound (phenol) with ammonia and hydrogen in the presence of a catalyst at elevated temperature and preferably under superatmospheric pressure, wherein the reaction is carried out in the presence of a supported palladium catalyst which contains a basic substance and/or an element from group 1B, 2B or 7B of the periodic table, iron, cobalt or nickel, the preparation of cycloaliphatic compounds using a hydrogen partial pressure of at least 20 bars or a total pressure of at least 50 bars according to European Patent 53,817 being excluded.

2. A process as claimed in claim 1, wherein the catalyst contains, as the basic substance, an oxide, hydroxide or carbonate of an alkali metal or of an alkaline earth metal.

3. A process as claimed in claim 1, wherein the catalyst contains, as the basic substance, an oxide, hydroxide or carbonate of a rare earth metal.

4. A process as claimed in claim 2, wherein the catalyst contains a magnesium compound.

5. A process as claimed in claim 3, wherein the catalyst contains a praseodymium/neodymium compound or a cerium compound.

**Revendications**

1. Procédé pour la préparation d'amines cycloaliphatiques et/ou aromatiques de formule générale $RNH_2$, dans laquelle R représente un radical aryle ou cycloalkyle éventuellement substitué ou à noyaux condensés, par réaction de composés hydroxylés aromatiques correspondants (phénols) avec l'ammoniac et l'hydrogène en présence d'un catalyseur, à température élevée et, de préférence, sous pression élevée, caractérisé en ce qu'on mène la réaction en présence d'un catalyseur au palladium fixé sur support, qui contient une substance basique et/ou un élément du groupe 1B, 2B, 7B du système périodique, du fer, du cobalt ou du nickel, la préparation de composés cycloaliphatiques avec utilisation d'une pression partielle d'hydrogène d'au moins 20 bars ou d'une pression totale d'au moins 50 bars suivant le brevet européen n° 53 817 étant exclue.

2. Procédé selon la revendication 1, caractérisé en ce que le catalyseur contient, en tant que substance basique, un oxyde, un hydroxyde ou un carbonate d'un métal alcalin ou d'un métal alcalinoterreux.

3. Procédé selon la revendication 1, caractérisé en ce que le catalyseur contient, en tant que substance basique, un oxyde, un hydroxyde ou un carbonate d'un métal des terres rares.

4. Procédé selon la revendication 2, caractérisé en ce que le catalyseur contient un composé du magnésium.

5. Procédé selon la revendication 3, caractérisé en ce que le catalyseur contient un composé du praséodyme/néodyme ou du cérium.